(19) Europäisches Patentamt
European Patent Office
Office européen des brevets

(11) EP 2 336 284 A2

(12) EUROPEAN PATENT APPLICATION

(43) Date of publication:
**22.06.2011 Bulletin 2011/25**

(21) Application number: **10194195.3**

(22) Date of filing: **01.04.2009**

(51) Int Cl.:
*C11D 3/22* (2006.01) *C11D 3/386* (2006.01)
*C11D 17/00* (2006.01) *C11D 3/08* (2006.01)
*C11D 3/37* (2006.01)

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **01.04.2008 US 41443 P**

(62) Document number(s) of the earlier application(s) in accordance with Art. 76 EPC:
**09755434.9 / 2 260 139**

(71) Applicant: **The Procter & Gamble Company**
**Cincinnati, OH 45202 (US)**

(72) Inventors:
- **Oliver Richard Eugene**
  **Liberty Township, OH 45044 (US)**
- **Fahlbusch, Ramona Quintanilla**
  **West Chester, OH 45069 (US)**
- **Harrington, Roy Jerome**
  **Liberty Township, OH 45011 (US)**
- **Keith, Brian lee**
  **Hamersville, OH 45130 (US)**
- **Song, Brian Xiaoqling**
  **Mason, OH 45040 (US)**

(74) Representative: **Howard, Phillip Jan**
**Procter & Gamble**
**Technical Centres Limited**
**Whitley Road**
**Longbenton**
**Newcastle upon Tyne NE12 9TS (GB)**

Remarks:
This application was filed on 08-12-2010 as a divisional application to the application mentioned under INID code 62.

## (54) Gel automatic dishwashing detergent composition

(57) A detergent composition having a desired rheology formulated from a thickener system of xanthan gum and either an associative thickener or a soluble silicate; one or more enzymes; an enzyme stabilizer; and water having a pH from 7.0 to 10.

EP 2 336 284 A2

## Description

### FIELD OF THE INVENTION

The present composition relates to gel automatic detergent composition having xanthan gum, a co-thickener, enzymes and an enzyme stabilization system having the specified rheology.

### BACKGROUND OF THE INVENTION

Consumers of gel automatic dishwashing detergents require not only cleaning performance, but it is also required that the gel detergents behave in a desirable way while being dispensed into a machine from a container and then behave in a somewhat different way once the detergent is dispensed into a machine for acceptance of the detergent by consumers. If a gel detergent is too thin, that is, it exhibits rheological properties more similar to water, and then consumers will perceive that the gel detergent has diminished cleaning benefits and will reject the gel detergent. Conversely, if the gel detergent is too thick, that is, it exhibits rheological properties more similar to those of toothpaste, and then consumers will perceive that the gel detergent does not dissolve completely in wash nor completely dispense from the bottle and that they do not receive full value from their purchase. If the gel detergent lacks consistency of phase; that is, if the gel detergent exhibits distinctly different phases such as the appearance of a thin layer of liquid about a more viscous bulk fluid, much like synereis, for example the collection of whey on the surface of yogurt, then consumers will reject the gel detergent having perceived it of being of poor quality.

A gel automatic detergent composition will react to a new component or respond to a changed level of current components is not predictable, even to people who formulate such compositions.

Limitations on the use of phosphate builders in cleaning compositions, such as gel automatic detergent compositions, alter the viscosity of such gel automatic detergent compositions. Xanthan gum has been utilized as a thickener in gel automatic dishwashing compositions containing enzymes in US 6,835,703 at a pH of lower than 7. However, the enzymes in such a system have relatively poor stability and therefore require an enzyme stabilization system; additionally, it has been found that such compositions in US 6,835,703 result in solidified gels at a pH of 8.5. Enzymes maybe destabilized by unfolding of the three-dimensional structure of the enzyme or by enzyme breaking down. Common destabilizers include polar solvents like of water or other solvents, microbial attack, electrolytes, charged surfactant, temperature and extreme pH. Stabilizers are added to rigidify the structure of the enzymes include boric acid, glycols, small organic acids, and calcium chloride. In addition, proteases have a tendency to attack themselves and other enzymes, causing autolysis and proteolysis in the formulation.

Some traditional enzyme stabilization systems include boric acid, borax (sodium tetraborate decahydrate) and alkali metal borates. However, the interaction of these materials with xanthan gum causes the gel automatic detergent composition to solidify and have an undesired rheology. See WO 93/21299.

US 2005/0234318 Al discusses the use of water-miscible organic solvents and soluble calcium salts as stabilizing aids for enzymes. WO 2007/141527 discusses the use of one or more divalent metal compounds or salts and one or more non-ionic surfactant as a stabilization system for enzymes. However, the exemplified formulations do not appear to have the desired rheology or the enzyme stability according to the testing further defined herein.

Therefore, there arises a need for a gel automatic detergent composition utilizing xanthan gum as a thickener which exhibits a consumer preferred rheology, results in a smooth, creamy consistency and having a stabilized enzyme system.

### SUMMARY OF THE INVENTION

A detergent composition comprising: (a) a thickener system comprising: (i) xanthan gum; (ii) an associative thickener or a soluble silicate (b) one or more enzymes; (c) enzyme stabilization; and (d) water wherein the composition has a pH from 8.5 to 10 and a specified rheology of viscosity at 1 $sec^{-1}$ of from about 9,000 to about 26,000 centipoise; viscosity at 150 $sec^{-1}$ from about 100 to about 1,300 centipoise; K of from about 9.0 to about 26.00 Pascal Sec; n less than 1.0; and yield stress greater than 2.0 Pascal.

### DETAILED DESCRIPTION OF THE INVENTION

Ideally, gel automatic detergent compositions should be a non-Newtonian fluid which exhibits shear-thinning properties and have relatively high yield values. At a resting state, the gel automatic detergent composition should have relatively high viscosity. Stability is likewise of primary importance, i.e., there should be no significant evidence of phase separation or leaking after long standing. The gel automatic detergent composition, upon application of a shear force, should dispense easily from a container having a dispensing orifice giving a consistent flow rate and therefore volume

of composition being dispensed.

Once the gel automatic detergent composition is dispensed into a machine, into perhaps a dispensing cup or into a machine with an automatic dosing mechanism, the composition should then again return to its initial resting state and relatively high viscosity.

Next, the gel automatic detergent composition should behave under washing conditions such that the composition can be dispensed or dispersed from the dispensing cup or the automatic dosing mechanism.

"Tableware" means any type of dishware, glassware, cookware, and/or silverware, including, but not limited to, those made from glass, plastic, ceramic, metal, wood, porcelain, etc., as well as, any type of silverware which includes all types made from metal, plastic, wood, glass, ceramic, porcelain, etc. Tableware can include, but is not limited to, cooking and eating utensils, dishes, cups, bowls, glasses, silverware, pots, pans and similar surfaces.

Thickener System

The thickener system for use in the compositions herein, comprises xanthan gum or similar material and a co-thickener such as an associative polymer or water soluble silicates. The thickener system may comprise from about 0.1 wt% to about 15 wt% by weight of the composition.

Suitable thickening agents are viscoelastic, thixotropic thickening agents. The viscoelastic, thixotropic thickening agent in the compositions of the present invention is from about 0.1 % to about 10%, preferably from about 0.25% to about 8%, most preferably from about 0.5% to about 5%, by weight of the detergent composition.

Suitable thickeners which can be used in this composition include natural gums, such as xanthan gum, locust bean gum, guar gum, and the like. In one embodiment, xanthan gums are utilized. Xanthan gums are biopolysaccharides and suitable xanthan gums include, without limitation, products sold by Kelco Corporation under the trade names KELTROL®, such as KETROL® RD and KELTROL CG-SFT and KELZAN® as well as products sold by Rhodia under the trade names RHODIPOL®, RHODIGEL® and RHODICARE®, such as RHODICARE® T.

Co-Thickeners

The composition may comprise either a soluble silicate or an associative thickener to address any texture issues that may arise with the use of a xanthan gum thickener.

Soluble Silicate

The present gel automatic detergent composition may comprise water-soluble silicates. Water-soluble silicates herein are any silicates which are soluble to the extent that they do not adversely affect spotting/filming characteristics of the gel detergent composition. Aluminosilicate builders can be used in the present compositions though are not preferred for automatic dishwashing detergents.

The soluble silicate is typically used in an amount of about 0.4% to about 4.0% by weight; more preferably is present in an amount of about 0.75% to about 3% by weight and most preferably present in an amount of about 1% to about 2% by weight, based on the total weight of the composition.

Associative Thickener

The associative thickener is typically an addition polymer of three components: an alpha-beta-monoethylenically unsaturated monocarboxylic acid or dicarboxylic acid of from 3 to 8 carbon atoms such as acrylic acid or methacrylic acid to provide water solubility, a monoethylenically unsaturated copolymerizable monomer lacking surfactant capacity such as methyl acrylate or ethyl acrylate to obtain the desired polymer backbone and body characteristics, and a monomer possessing surfactant capacity which provides the pseudo plastic properties to the polymer and is the reaction product of a monoethylenically unsaturated monomer with a nonionic surfactant compound wherein the monomer is copolymerizable with the foregoing monomers such as the reaction product of methacrylic acid with a monohydric nonionic surfactant to obtain a monomer such as $CH_3(CH_2)_{15-17}(OCH_2CH_2)e\ OOCC(CH_3)=CH_2$ where "e" has an average value of about 10 or 20. Optionally, up to about 2.0% of a polyethylenically unsaturated monomer sloth as ethylene glycol diacrylate or dimethacrylate or divinylbenzene can be included if a higher molecular weight polymer is desired.

Additional associative thickeners include maleic anhydride copolymers reacted with nonionic surfactants such as ethoxylated $C_{12}$-$C_{14}$ primary alcohol available under the trade name Surfonic L Series from Texaco Chemical Co. and Gantrez AN-119 from ISP.

The associative thickeners include $C_{10}$-$C_{22}$ alkyl groups in an alkali-soluble acrylic emulsion polymer such as those available under the trademark "Acusol®" from Rohm and Haas Co. of Philadelphia, Pa. The most preferred associative thickeners are Acusol. RTM. 820 ("820") and 1206A ("1206A"). Acusol® 820 is a 30.0% active emulsion

polymer of 40. 0% methacrylic acid, 50% ethyl acrylate and 10.0% stearyl oxypoly ethyl methacrylic emulsion polymer having approximately 20 moles of ethylene oxide. Acusol® 1206A is a 30% active emulsion polymer with 44% methacrylic acid, 50% ethyl acrylate and 6% stearyl methacrylate polymer having about 10 moles of ethylene oxide. These polymers are described in U.S. 4,351,754 to Dupre.

The associative thickener is typically used in an amount of about 0.01 % to about 1.0% by weight; more preferably is present in an amount of about 0.05% to about 0.5% by weight and most preferably present in an amount of about 0.1% to about 0.3% by weight, based on the total weight of the composition.

As used herein, enzymes means any enzyme having a cleaning, stain removing or otherwise beneficial effect in a gel detergent composition. Preferred enzymes are hydrolases such as proteases, amylases and lipases. Highly preferred for automatic dishwashing are amylases and/or proteases, including both current commercially available types and improved types. Enzymes are normally incorporated in the instant detergent compositions at levels sufficient to provide a "cleaning-effective amount". The term "cleaning-effective amount" refers to any amount capable of producing a cleaning, stain removal or soil removal effect on substrates such tableware.

The compositions herein can comprise: from about 0.001% to about 10%, preferably from about 0.005% to about 8%, most preferably from about 0.01% to about 6%, by weight of an enzyme stabilizing system.

The purpose of an enzyme stabilizing system is to protect the enzymes in the composition between the time the composition is manufactured and the time the composition is use. It is preferred that the enzyme activity remains between about 60% and 100%, more preferably between about 70% and 100%, more preferably about 80% and 100%. In one embodiment, the stabilized enzyme is a protease and the enzyme activity is of such protease.

The enzyme activity may be testing utilizing the following method:

A 4 oz. jar of the product ("PRODUCT") for enzyme stability testing are held at 90°F for 24 days (4 weeks) and is tested at 7 day (1 week) intervals for enzyme activity. Activity is measured against a calibration curve created from testing solutions created from an enzyme-free PRODUCT having premeasured amounts of the enzyme added.

The testing solutions are created such that the mg of active enzyme per 100 g enzyme-free PRODUCT is from 1 to 7 mg active enzyme per 100 g enzyme-free PRODUCT. Add 60 microliters of the testing solutions is placed into individual curvets. Add 1.5 mL of a Succinyl - L-ALA - L - ALA - L - PRO - PHE[1] - p - nitroanilide ("PNA") substrate to each curvet and mix by inversion.

[1] alanine, alanine, proline, phenylalanine

A PNA substrate can be prepared add 200 microliter of PNA solution (Weigh 0.250 ± 0.005 g of para nitroanilide into a small glass vial and add by pipette, 2 ml of DMSO. Swirl to mix) to 20 ml of Tris buffer and mix.

A Tris buffer may be prepared by measuring into a liter beaker: 12.0 g of Tris (hydroxymethyl) aminomethane; 1.5 g of $CaCl_2.2H_2O$ and 5.0 g of sodium thiosulfate. Add 800 - 900 ml deionized water and stir to dissolve. Adjust the pH to 8.3 with concentrated hydrochloric acid and make up to 1 litre with deionized water.

Use a spectrophotometer, such as a Kontron Uvikon 930, set the following parameters to measure the 1-7 mg active enzyme/100 g F.P. samples:

| | |
|---|---|
| - measuring program | Self-test; Kinetics method |
| -water bath temp. | 37°C |
| - Curvet lightpath | 0.5 cm |
| - Wavelength | 403.0 nm |
| - Incubation time | 0 sec |
| - Reading interval | 60 sec |
| - Integration time | 1 sec |
| - Number of readings | 30 |
| - Factor | 1.00 |

And a blank enzyme-free PRODUCT as a reference. Plot out the delta absorbance between min. 5 and min. 10. The PRODUCT is then tested by adding 60 microliters of the PRODUCT into curvets. Add 1.5 mL of a PNA substrate to each curvet and mix by inversion. Utilizing the parameters above for the spectrophotometer and the calibration curve, the enzyme activity can be determined.

In one embodiment, after 4 weeks at 90°F, a minimum of 60% enzyme activity of the PRODUCT compared to

the initial PRODUCT enzyme activity (on day 1 of testing). In another embodiment, after 4 weeks at 90°F, from about 60% to 100% enzyme activity of the PRODUCT compared to the initial PRODUCT enzyme activity (on day 1 of testing). In another embodiment, after 4 weeks at 90°F, from about 70% to 100% enzyme activity of the PRODUCT compared to the initial PRODUCT enzyme activity (on day 1 of testing).

The enzyme stabilizing system can be any stabilizing system which can be compatible with the detersive enzyme and with the xanthan gum thickener - thereby excluding boric acid, borax (sodium tetraborate decahydrate) and alkali metal borates. Such stabilizing systems can comprise calcium ion, glycerin, propylene glycol, short chain carboxylic acid and mixtures thereof.

In one embodiment, the enzyme stabilization system includes a phenyl boronic acid derivative enzyme stabilizer of the following formula (I):

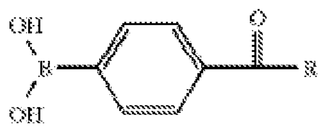

Formula (I)

wherein R of formula (I) is selected from the group consisting of hydrogen, hydroxy, $C_1$-$C_6$ alkyl, substituted $C_1$-$C_6$ alkyl, $C_1$-$C_6$ alkenyl and substituted $C_1$-$C_6$ alkenyl.

A preferred embodiment provides a liquid composition comprising an enzyme and a phenyl boronic acid derivative enzyme stabilizer of the formula disclosed above, wherein R of formula (I) is a $C_1$-$C_6$ alkyl, in particular wherein R of formula (I) is $CH_3$, $CH_3CH_2$ or $CH_3CH_2CH_2$, or wherein R of formula (I) is hydrogen. A preferred compound is 4-Formyl-Phenyl-Boronic Acid.

In another embodiment, the enzyme stabilization system is a tripeptide enzyme inhibitor. By tripeptide enzyme inhibitor, it is meant a compound that comprises three amino acids or their derivatives that may be substituted or unsubstituted. One illustrative tripeptide enzyme inhibitor has the formula (II):

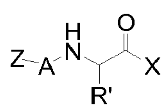

Formula (II)

In formula (II), A is a diamino acid moiety, more specifically the diamino acid moiety is a combination of two amino acids selected from alanine (Ala), arginine (Arg), asparagine (Asn), aspartic acid (Asp), cysteine (Cys), glutamine (Gln), glutamic acid (Glu), glycine (Gly), histidine (His), homophenylalanine (HPhe), isoleucine (Ile), leucine (Leu), lysine (Lys), methionine (Met), phenyalanine (Phe), phenylglycine (PGly), proline (Pro), serine (Ser), threonine (Thr), tryptophan (Trp), tyrosine (Tyr) and valine (Val).

In one embodiment of formula (II), A comprises two of alanine, glycine, leucine, valine, isoleucine, proline, lysine, phenylalanine, homophenylalanine, phenylglycine, tryptophan, glycine, arginine, methionine and combinations thereof, even more specifically still, valine and alanine.

The diamino acid moiety may be any suitable optical isomer, that is, the diamino acid moiety may be optically active in either the L or D configuration or combinations thereof, be optically inactive, or be a racemic mixture. Similarly, the individual amino acids that comprise the diamino acid moiety and/or the reversible peptide protease inhibitor may be optically active in either the L or D configuration or combinations thereof, or be optically inactive, or be a racemic mixture.

In formula (II), X is H, an electron withdrawing group and mixtures thereof. Non limiting examples of suitable electron withdrawing groups include, but are not limited to, $CF_2H$, $CH_2F$, $CF_2$-R CHF-R, $CO_2$-R $CH_2Cl$, substituted or unsubstituted imidazoles, substituted or unsubstituted thioamidazoles, substituted or unsubstituted benzimidazoles, and mixtures thereof, wherein R formula (II) is selected from the group consisting of linear or branched, substituted or unsubstituted $C_1$-$C_6$ alkyl; and linear or branched substituted or unsubstituted $C_4$-$C_8$ cycloalkyl moieties; and mixtures thereof.

In formula (II), Z is an N-capping moiety selected from:

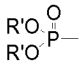, 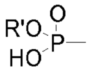, 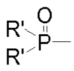, 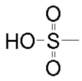, 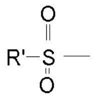, 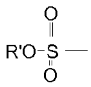, $S(R')_2-$, 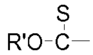, 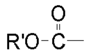, 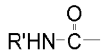, 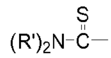

and mixtures thereof, more specifically,

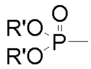, 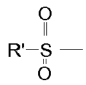, 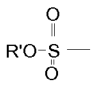, 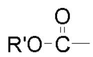, 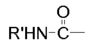, 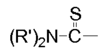

and mixtures thereof; and even more specifically,

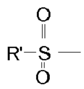, 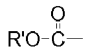

and mixtures thereof.

R' of formula (II) is independently selected from linear or branched, substituted or unsubstituted $C_1$ -$C_6$ alkyl; phenyl; linear or branched, substituted or unsubstituted $C_7$-$C_9$ alkylaryl; linear or branched substituted or unsubstituted $C_4$-$C_8$ cycloalkyl moieties; and mixtures thereof, more specifically linear or branched, $C_1$ -$C_6$ alkyl; phenyl; linear or branched, $C_7$-$C_9$ alkylaryl; and mixtures thereof, and even more specifically, linear or branched, $C_1$ -$C_6$ alkyl; linear or branched substituted or unsubstituted $C_5$-$C_9$ alkylheterocyclic; and mixtures thereof.

Illustrative non-limiting examples of suitable tripeptide enzyme inhibitor include:

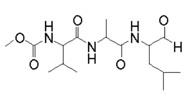, 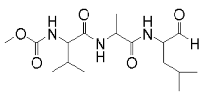, 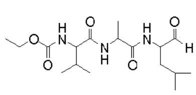, 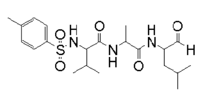, 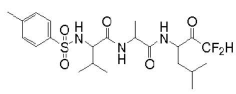,

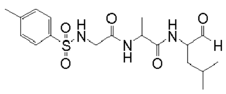, 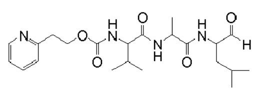, 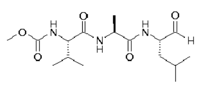,

and mixtures thereof.

The reversible peptide protease inhibitor may be made in any suitable manner. Illustrative examples of suitable process for the manufacture of the reversible peptide protease inhibitor may be found in U.S. Patent No. 6,165,966. pH adjusting components - The above gel automatic detergent composition comprises a neat pH in a range of desirably from about 8.0 to about 10.0, more preferably from about 8.5 to about 9.8. Preferably the neat pH can be less than about 10.0 for better enzyme stability, most preferably less than about 9.5. The pH adjusting components are desirably selected from sodium or potassium hydroxide, sodium or potassium carbonate, potassium silicate, sodium or potassium bicarbonate, and mixtures thereof. Sodium hydroxide or potassium hydroxide is the preferred ingredient for increasing the neat pH to within the above ranges. As used herein "neat pH" means the pH of the composition is tested without dilution in water of the composition.

## RHEOLOGY

Consumers experience thickness at two distinct times during their use of a gel automatic detergent composition. First, as a consumer dispenses the product from the bottle the fluid's resistance to flow through the bottle's opening will influence their perception of thickness. A low resistance will be perceived as thin and watery, while a high resistance will be perceived as too thick. This resistance to flow is a function of the fluid's viscosity at the shear rate applied to the fluid during dispensing by the consumer. A representative shear rate during dispensing may be 150 inverse seconds.

A second impression of thickness will be formed by the consumer upon inspection of the product as it appears in the dishwasher's dispensing cup. If the fluid mounds as it is dispensed and retains some of its shape in the cup, the

consumer will be accepting of the product. However, if the fluid readily forms a flat surface in the cup, much like water would, the consumer will find this product to be thin and reject the gel detergent as having diminished cleaning performance. The yield stress of the fluid can be correlated with this particular fluid behavior.

A third point, while not necessarily observable by the consumer at the time of dispensing occurs when the machine door is closed. The product must have a high enough viscosity to resist flow out of the closed main wash dispensing cup. The force of gravity will tend to pull the gel into the machine prematurely so that the gel automatic detergent composition would not be available during the main wash cycle; therefore, the consumer would experience a diminished performance from the gel detergent. A representative shear rate for the force of gravity on a fluid through a closed cup may be 1 inverse second.

Viscosity is a measure of the internal resistance to flow exhibited by a fluid in terms of the ratio of the shear stress to the shear rate. The yield value is an indication of the shear stress at which the gel strength is exceeded and flow is initiated.

A preferred method herein for characterizing a fluid's rheology is by using the Advanced Rheometer AR 2000 that employs Rheology Advantage software to control the rheometer and collect the data generated by the rheometer as it measures a fluid's responses to various forces applied to the fluid.

The data collected by the rheometer may then be evaluated using TA Data Analysis software provided by TA Instruments Thermal Analysis and Rheology to characterize the fluid's rheology. For rheology characterization, the rheometer is first calibrated per the manufacturers recommended methods using a specified tool; in this case, a 40 millimeter diameter stainless steel conical plate having a 2 degree slope.

After proper calibration, a small sample of the fluid is placed onto the instrument and the tool is placed at a specified gap between the tool and the measurement plate. The sample and the equipment are brought to temperature equilibrium at 25°C. Next, the rheometer measures the shear stress as the shear rate ramps up from 0.01 to 1.00 inverse seconds while recording 30 points per decade. The sample is held at a shear rate of 1 inverse second for a time of five seconds and the shear stress is measured each second for five seconds. Then, the rheometer measures the shear stress as the shear rate is ramped down from 1.00 to 0.01 inverse seconds while recording 30 points per decade.

The sample is allowed to rest for one minute to return to equilibrium. A shear at 0.25 inverse seconds is applied for five seconds. Next, the rheometer measures the shear stress as the shear rate ramps up from 0.25 to 150 inverse seconds while recording 30 points per decade. The sample is held at a shear rate of 150 inverse seconds for a time of five seconds and the shear stress is measured each second for five seconds. Then, the rheometer measures the shear stress as the shear rate ramps down from 150 to 0.25 inverse seconds while recording 30 points per decade.

The data collected by the rheometer may then be used to infer specific rheological parameters that can be correlated to consumer preferred gel automatic detergent composition rheology. One such set of parameters include the values of K and n. These values may be determined using the Power law equation.

Power Law Equation:

$$\text{Shear Stress} = \text{viscosity} * \text{Shear Rate}^{n}$$

The Power Law Equation is the simplest available method to predict the change in viscosity as a function of shear. Most non-Newtonian fluids may be described using this equation. The viscosity is replaced by a consistency coefficient, K. By definition, K will equal the viscosity at a shear rate of 1.0 inverse seconds. For shear thinning fluids, the index, n, will have a value less than 1. Values for K and n are commonly used to define the design requirements for equipment used in processing shear thinning fluids and may also be used to gauge the acceptance by consumers of a gel detergent.

A useful model for evaluating the yield stress of a shear thinning fluid is the Herschel-Bulkley equation.

Herschel-Bulkley equation:

$$\text{Shear Stress} = \text{Yield Stress} + \text{viscosity} * \text{Shear Rate}^{n}$$

Using the TA Data Analysis software, the data collected from the rheometer can be evaluated to predict Yield Stress, K and n values. Yield stress is calculated using the Herschel-Bulkley equation to evaluate the data from the down curve from 0.04 to 0.01 inverse seconds shear rates. K and n values are calculated using the Power Law equation to evaluate the data from the down curve from 1.0 to 0.04 inverse seconds shear rates.

The viscosity of the gel automatic detergent composition is at 1 $sec^{-1}$ of from about 9,000 to about 26,000

centipoise; viscosity at 150 $sec^{-1}$ from about 100 to about 1,300 centipoise; K of from about 9.0 to about 26.00 Pascal Sec; n less than 1.0; and yield stress greater than 2.0 Pascal.

In one embodiment, the gel automatic detergent composition viscosity of the gel automatic detergent composition is at 1 $sec^{-1}$ of from about 9,000 to about 10,000 centipoise; Viscosity at 150 $sec^{-1}$ from about 100 to about 200 centipoise; K of from about 9.0 to about 10.00 Pascal Sec; n less than 1.0; and yield stress greater than 2.0 Pascal.

In one embodiment, the gel automatic detergent composition viscosity of the gel automatic detergent composition is at 1 $sec^{-1}$ of from about 14,000 to about 26,000 centipoise; viscosity at 150 $sec^{-1}$ from about 450 to about 1,300 centipoise; K of from about 14.00 to about 26.00 Pascal Sec; n less than 1.0; and yield stress greater than 3.40 Pascal.

In one embodiment, the gel automatic detergent composition viscosity of the gel automatic detergent composition is at 1 $sec^{-1}$ of about 20,000 centipoise; viscosity at 150 $sec^{-1}$ 720 centipoise; K of 20.00 Pascal Sec; n is from 0.1 to 0.3; and yield stress greater than 12.00 Pascal.

Low-Foaming Nonionic Surfactant

Gel automatic detergent compositions of the present application comprise low foaming nonionic surfactants (LFNIs). LFNI can be present in amounts from about 0.1% to about 2%. LFNIs are most typically used in gel automatic detergents on account of the improved water-sheeting action (especially from glass) which they confer to the gel automatic detergents.

Preferred LFNIs include nonionic alkoxylated surfactants, especially ethoxylates derived from primary alcohols, and blends thereof with more sophisticated surfactants, such as the polyoxypropylene/polyoxyethylene/polyoxypropylene (PO/EO/PO) reverse block polymers. The PO/EO/PO polymer-type surfactants are well-known to have foam suppressing or defoaming action, especially in relation to common food soil ingredients such as egg.

In a preferred embodiment, the LFNI is an ethoxylated surfactant derived from the reaction of a monohydroxy alcohol or alkylphenol containing from about 8 to about 20 carbon atoms, excluding cyclic carbon atoms, with from about 6 to about 15 moles of ethylene oxide per mole of alcohol or alkyl phenol on an average basis.

A particularly preferred LFNI is derived from a straight chain fatty alcohol containing from about 16 to about 20 carbon atoms ($C_{16}$-$C_{20}$ alcohol), preferably a $C_{18}$ alcohol, condensed with an average of from about 6 to about 15 moles, preferably from about 7 to about 12 moles, and most preferably from about 7 to about 9 moles of ethylene oxide per mole of alcohol. Preferably the ethoxylated nonionic surfactant so derived has a narrow ethoxylate distribution relative to the average.

The LFNI can optionally contain propylene oxide in an amount up to about 15% by weight. Certain of the block polymer surfactant compounds designated PLURONIC® and TETRONIC® by the BASF-Wyandotte Corp., Wyandotte, Mich., are suitable in gel automatic detergents of the invention. Highly preferred gel automatic detergents herein wherein the LFNI is present make use of ethoxylated monohydroxy alcohol or alkyl phenol and additionally comprise a polyoxyethylene, polyoxypropylene block polymeric compound; the ethoxylated monohydroxy alcohol or alkyl phenol fraction of the LFNI comprising from about 20% to about 80%, preferably from about 30% to about 70%, of the total LFNI.

LFNIs which may also be used include a $C_{18}$ alcohol polyethoxylate, having a degree of ethoxylation of about 8, commercially available SLF18 from Olin Corp.

Dispersant Polymer

Preferred compositions herein may additionally contain a dispersant polymer typically in the range from 0 to about 25%, preferably from about 0.5% to about 20%, more preferably from about 1% to about 7% by weight of the gel automatic detergents.

One dispersant polymer suitable for use in the present composition includes an ethoxylated cationic diamine comprising the formula (III):

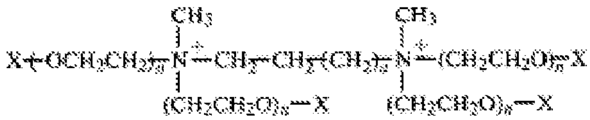

Formula (III)

wherein X of formula (III) is a nonionic group selected from the group consisting of H, $C_1$-$C_4$ alkyl or hydroxyalkyl ester

or ether groups, and mixtures thereof; n is at least about 6; and a is from 0 to 4 (e. g. ethylene, propylene, hexamethylene). For preferred ethoxylated cationic diamines, n of formula (III) is at least about 12 with a typical range of from about 12 to about 42. See US 4,659,802 for further information regarding the ethoxylated cationic diamines.

Further suitable dispersant polymers suitable for use herein are illustrated by formula (IV)

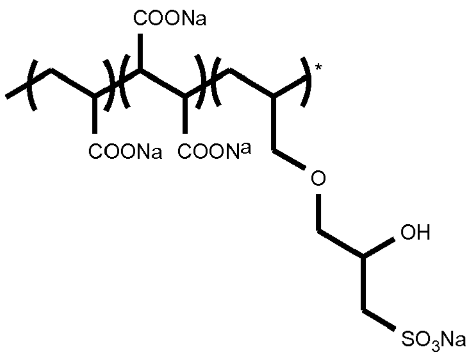

Formula (IV)

Formula IV is an Acrylic acid (AA), maleic acid (MA) and sodium 3-allyloxy-2-hydroxy-1-propanesulfonate (HAPS) copolymer, preferably comprising about 45 wt% by weight of the polymer of AA, about 45 wt% by weight of the polymer of MA and about 10 wt% by weight of the polymer of HAPS. Molecular weight may be from about 8000 to about 15000. In one embodiment, formula (IV) comprises a molecular weight of about 8000 to about 8500. In another embodiment formula (IV) comprises a molecular weight of about 12500 to about 13300. Salts of formula (IV) may be selected from any water soluble salt such as sodium or potassium salt.

Further suitable dispersant polymers suitable for use herein are illustrated by the film-forming polymers. Suitable for use as dispersants herein are co-polymers synthesized from acrylic acid, maleic acid and methacrylic acid such as ACUSOL® 480N supplied by Rohm & Haas and polymers containing both carboxylate and sulphonate monomers, such as ALCOSPERSE® polymers (supplied by Alco). In one embodiment an ALCOSPERSE® polymer sold under the trade name ALCOSPERSE® 725, is a co-polymer of Styrene and Acrylic Acid with the following structure shown in formula (IV):

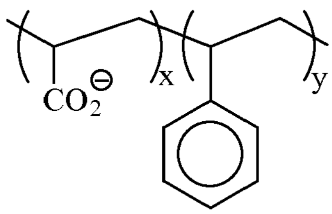

Formula (V)

Wherein the x : y ratio of formula (V) is from about 60 : 40 or about 50 : 50 and the polymer having a molecular weight about 8000.

In certain embodiments, a dispersant polymer may be present in an amount in the range from about 0.01% to about 25%, or from about 0.1% to about 20%, and alternatively, from about 0.1 % to about 7% by weight of the composition.

Further suitable dispersant polymers include polyacrylic phosphono end group polymers or acrylic-maleic phosphono end group copolymers for use herein are according to the general formula (V): $H_2PO_3$-($CH_2$-CHCOOH)$_n$-(CH-COOH-CHCOOH)m- wherein n of formula (VI) is an integer greater than 0, m of formula (VI) is an integer of 0 (for polyacrylic polymers) or greater (for acrylic-maleic copolymers) and n and m of formula (VI) are integers independently selected to give a molecular weight of the polymer of between 500 and 200,000, preferably of between 500 and 100,000, and more preferably between 1,000 and 50,000. For polyacrylates, m of formula (VI) is zero. Suitable polyacrylic phosphono end group polymers or acrylic-maleic phosphono end group copolymers for use herein are available form Rohm &

Haas under the tradenames ACUSOL® E 420 or 470 or 425. In one embodiment Acusol 425N is utilize, Acusol 425N is an acrylic-maleic (ratio 80/20) phosphono end group copolymers available from Rohm & Haas

Particularly preferred dispersant polymers are low molecular weight modified polyacrylate copolymers. Such copolymers contain as monomer units: a) from about 90% to about 10%, preferably from about 80% to about 20% by weight acrylic acid or its salts and b) from about 10% to about 90%, preferably from about 20% to about 80% by weight of a substituted acrylic monomer or its salt and have the general formula (VII):--[(C($R^2$)C($R^1$)(C(O)O$R^3$)]-- wherein the incomplete valencies inside the square braces of formula (VII) are hydrogen and at least one of the substituents $R^1$, $R^2$ or $R^3$ of formula (VII), preferably $R^1$or $R^2$ of formula (VII), is a 1 to 4 carbon alkyl or hydroxyalkyl group, $R^1$or R2 of formula (VII) can be a hydrogen and R3 of formula (VII) can be a hydrogen or alkali metal salt. Most preferred is a substituted acrylic monomer wherein R1 of formula (VII) is methyl, $R^2$ of formula (VII) is hydrogen and $R^3$ of formula (VII) is sodium.

The low molecular weight polyacrylate dispersant polymer preferably has a molecular weight of less than about 15,000, preferably from about 500 to about 10,000, most preferably from about 1,000 to about 5,000. The most preferred polyacrylate copolymer for use herein has a molecular weight of 3500 and is the fully neutralized form of the polymer comprising about 70% by weight acrylic acid and about 30% by weight methacrylic acid.

Other thickeners

In addition to the xanthan gum thickener, other thickeners may be utilized. Suitable are various carboxyvinyl polymers, homopolymers and copolymers are commercially available from B. F. Goodrich Company, New York, N.Y., under the trade name CARBOPOL®. These polymers are also known as carbomers or polyacrylic acids. Carboxyvinyl polymers useful in formulations of the present invention include CARBOPOL 910 having a molecular weight of about 750,000, CARBOPOL® 941 having a molecular weight of about 1,250,000, and CARBOPOL 934 and 940 having molecular weights of about 3,000,000 and 4, 000,000, respectively. More preferred are the series of CARBOPOL® which use ethyl acetate and cyclohexane in the manufacturing process, for example, CARBOPOL® 981, 2984, 980, and 1382.

Suitable additional thickeners include polycarboxylate polymers of the invention are non-linear, water-dispersible, polyacrylic acid cross-linked with a polyalkenyl polyether and having a molecular weight of at lease 750,000, preferably from about 750,000 to about 4,000,000. Suitable examples of these polycarboxylate polymers for use in the present invention are SOKALAN PHC-25®, a polyacrylic acid available from BASF Corporation and the POLYGEL® series available from 3-V Chemical Corporation. Mixtures of polycarboxylate polymers may also be used.

Semi-synthetic thickeners such as the cellulosic type thickeners: hydroxyethyl and hydroxymethyl cellulose (ETHOCEL® and METHOCEL® available from Dow Chemical) can also be used. Mixtures Inorganic clays (e.g. aluminum silicate, bentonite, fumed silica) are also suitable for use as a thickener herein. The preferred clay thickening agent can be either naturally occurring or synthetic. A suitable synthetic clay is the one disclosed in the U.S. 3,843,598. Naturally occurring clays include some smectite and attapulgite clays as disclosed in U.S. 4,824, 590.

Builders

Detergent builders suitable to be included in the compositions herein to assist in controlling mineral hardness and dispersancy, with the exception of phosphate builders. Inorganic as well as organic builders can be used. One embodiment of the present invention relates to a gel detergent composition, wherein the builder can be selected from the group consisting of carbonate builders, polycarboxylate compounds, citrate, methyl glycine diacetic acid and/or salts thereof, glutamatic diacetic acid and/or salts thereof and mixtures thereof.

Examples of carbonate builders are the alkaline earth and alkali metal carbonates as disclosed in German Patent Application No. 2,321,001 published on November 15, 1973. Various grades and types of sodium carbonate and sodium sesquicarbonate can be used, certain of which are particularly useful as carriers for other ingredients, especially: detersive surfactants.

Organic detergent builders suitable for the purposes of the present invention include, but are not restricted to, a wide variety of polycarboxylate compounds.

Other useful detergency builders include the ether hydroxypolycarboxylates, copolymers of maleic anhydride with ethylene or vinyl methyl ether, 1, 3, 5-trihydroxy benzene-2, 4, 6-trisulphonic acid, and carboxymethyloxysuccinic acid, the various I alkali metal, ammonium and substituted ammonium salts of polyacetic acids such as ethylenediaminetetraacetic acid and nitrilotriacetic acid, as well as polycarboxylates such as mellitic acid, succinic acid, oxydisuccinic acid, polymaleic acid, benzene 1,3,5-tricarboxylic acid, carboxymethyloxysuccinic acid, and soluble salts thereof.:

Citrate builders, e.g., citric acid and soluble salts thereof (particularly sodium salt), builders suitable herein due to their availability from renewable resources and their biodegradability.

Methyl glycine diacetic acid and/or salts thereof (MGDA) may also be utilized as builders in the present composition. A preferred MGDA compound is a salt of methyl glycine iacetic acid Suitable salts include the diammonium salt, the dipotassium salt and, preferably, the disodium salt.

Glutamatic diacetic acid and/or salts thereof (GLDA) may also be utilized as builders in the present composition. A preferred GLDA compound is a salt of glutamic diacetic acid. Suitable salts include the diammonium salt, the dipotassium salt and, preferably, the disodium salt.

Perfume - Non-Blooming Perfumes-Perfumes and perfumery ingredients useful in the present compositions and processes comprise a wide variety of natural and synthetic chemical ingredients, including, but not limited to, aldehydes, ketones, esters, and the like.

Metal-Protecting Agent - The present gel detergent composition compositions can contain one or more material care agents which are effective as corrosion inhibitors and/or anti-tarnish aids.

Chelating Agents - The compositions herein can also optionally contain one or more transition-metal selective sequestrants, "chelants" or "chelating agents", e.g., iron and/or copper and/or manganese chelating agents. Chelating agents suitable for use herein can be selected from the group consisting of aminocarboxylates, polyfunctionally-substituted aromatic chelating agents, and mixtures thereof. Commercial chelating agents for use herein include the BEQUEST series, and chelants from Monsanto, DuPont, and Nalco, Inc.

Organic Solvent - One embodiment of the present invention relates to a gel detergent composition comprising an organic solvent selected from the group consisting of low molecular weight aliphatic or aromatic alcohols, low molecular weight alkylene glycols, low molecular weight alkylene glycol ethers, low molecular weight esters, low molecular weight alkylene amines, low molecular weight alkanolamines, and mixtures thereof.

Any adjunct ingredient in any amount may be used in the gel detergent composition. For example, adjunct ingredients may be selected from the group consisting of nanoparticles, functionalized surface molecules, polymers, surfactants, co-surfactants, metal ions, proteins, dyes, acids, optical brighteners, colorants, filler salts, hydrotropes, preservatives, anti-oxidants, germicides, fungicides, color speckles, solubilizing agents, carriers and mixtures thereof.

Container

The gel detergent composition may be contained in a suitable container to facilitate transportation and dispensing of the composition. In one embodiment, the container comprises a dispensing orifice having an approximate diameter between about 5 mm and 10 mm. The shape of the dispensing orifice may be selected from circular, diamond shaped or tear-drop shaped. Examples of suitable diamond shaped or tear-drop shaped orifices may be seen in US 2005/0194398, US 2006/0016836 and US 2006/0186077.

Examples

| | A | B | c |
|---|---|---|---|
| Citric Acid (50%) | 12.5400 | 2.68 | 2.68 |
| MGDA (39%) | -- | 12.82 | -- |
| GLDA (45%) | -- | -- | 11.11 |
| Sodium silicate (46%) | -- | 3.75 | 3.75 |
| Xanthan Gum | 1.1000 | 0.80 | 0.80 |
| Acusol 820 (30%) | 0.3400 | -- | -- |
| Sodium Hydroxide (50%) | 9.46 | -- | -- |
| Sodium Benzoate soln (33%) | 0.6100 | 0.6100 | 0.6100 |
| Poly-tergent SLF18 | 1.0000 | 1.00 | 1.00 |

(continued)

| | A | B | c |
|---|---|---|---|
| Calcium Chloride (25%) | 1.8000 | 1.80 | 1.80 |
| Sodium bicarbonate | -- | 10.00 | 10.00 |
| Ethoxylated cationic diamine | -- | 2.0 | 2.00 |
| Acusol 425N (49.4%) | 3.0000 | -- | -- |
| sulfonated acrylic/maleic polymer[1] | -- | 5.0 | 5.0 |
| Proxel GXL | 0.0500 | 0.05 | 0.05 |
| phenyl boronic acid derivative[2] | 0.0100 | 0.02 | -- |
| tripeptide enzyme inhibitor[3] | -- | -- | 0.0025 |
| Protease liquid | 0.2400 | 0.60 | 0.60 |
| alpha-amylase liquid | 0.0700 | 0.17 | 0.17 |
| ethanol | -- | -- | 0.30 |
| Water, dyes, perfume | balance | balance | balance |

[1] such as that in formula (IV)
[2] such as that in formula (I)
[3] such as that in formula (II)

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

**1.** A detergent composition comprising:

(a) from 0.1 wt% to 15 wt% of a thickener system comprising:

(i) xanthan gum; and
(ii) an associative thickener or a soluble silicate;

(b) one or more enzymes;
(c) from 0.001 wt% to 10 wt% of an enzyme stabilizer excluding boric acid, sodium tetraborate decahydrate and alkali metal borates; and
(d) water;
wherein the composition has a pH from 8.5 to 10 and a specified rheology of viscosity at 1 $sec^{-1}$ of from 9,000 to 26,000 centipoise; viscosity at 150 $sec^{-1}$ from 100 to 1,300 centipoise; K of from 9.0 to 26.00 Pascal Sec; n less than 1.0; and yield stress greater than 2.0 Pascal.

**2.** The detergent composition of claim 1 wherein the composition further comprises from 0.1 wt% to 2 wt% of low foaming nonionic surfactants.

**3.** The detergent composition of claim 1 or 2 wherein the associative thickener is $C_{10}$-$C_{22}$ alkyl groups in an alkali-soluble acrylic emulsion polymer.

**4.** The detergent composition of any one of claims 1 to 3 wherein the one or more enzymes are selected from proteases, alpha-amylases and mixtures thereof.

**5.** The detergent composition of any one of claims 1 to 4 wherein the enzyme stabilizer is selected from the group

consisting of:

(a) a phenyl boronic acid derivative enzyme stabilizer of the following formula (I):

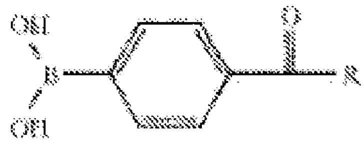

Formula (I)

wherein R is selected from the group consisting of hydrogen, hydroxy, $C_1$-$C_6$ alkyl, substituted $C_1$-$C_6$ alkyl, $C_1$-$C_6$ alkenyl and substituted $C_1$-$C_6$ alkenyl; and
(b) a tripeptide enzyme inhibitor.

**6.** The detergent composition of any one of claims 1 to 5 wherein the composition further comprises a dispersant polymer.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6835703 B
- WO 9321299 A
- US 20050234318 A1
- WO 2007141527 A
- US 4351754 A, Dupre
- US 6165966 A
- US 4659802 A
- US 3843598 A
- US 4824590 A
- DE 2321001
- US 20050194398 A
- US 20060016836 A
- US 20060186077 A